# EUROPÄISCHE PATENTSCHRIFT

(11) **EP 2 751 618 B1**
(45) Veröffentlichungstag und Bekanntmachung des Hinweises auf die Patenterteilung: **29.05.2019**
(21) Anmeldenummer: 13720820.3
(22) Anmeldetag: 11.04.2013
(51) Int. Cl.: G03F 7/00, G03F 7/029, A61K 6/083, A61C 13/00, G03F 7/027

(54) **KOMPOSITHARZ-ZUSAMMENSETZUNG UND DEREN VERWENDUNG, VERFAHREN ZUR HERSTELLUNG DENTALER BAUTEILE MITTELS STEREOLITHOGRAPHIE**
COMPOSITE RESIN COMPOSITION AND USE OF IT, METHOD FOR PRODUCING DENTAL COMPONENTS BY MEANS OF STEREO-LITHOGRAPHY
COMPOSITION DE RÉSINE COMPOSITE ET SON UTILISATION, PROCÉDÉ DE FABRICATION DE COMPOSANTS DENTAIRES PAR STÉRÉOLITHOGRAPHIE

(30) Priorität: 11.04.2012 EP 12163824
(43) Veröffentlichungstag der Anmeldung: 09.07.2014
(73) Patentinhaber: Ivoclar Vivadent AG, 9494 Schaan (LI); Technische Universität Wien, 1040 Wien (AT)
(72) Erfinder: WACHTER, Wolfgang, FL-9494 Schaan (LI); EBERT, Jörg, CH-9470 Buchs (CH); VOSER, Dieter, FL-9494 Schaan (LI); MOSZNER, Norbert, FL-9495 Triesen (LI); RHEINBERGER, Volker, FL-9490 Vaduz (LI); STAMPFL, Jürgen, A-1050 Wien (AT)
(74) Vertreter: Uexküll & Stolberg
(86) Internationale Anmeldenummer: PCT/EP2013/057635
(87) Internationale Veröffentlichungsnummer: WO 2013/153183

(56) Entgegenhaltungen:
- WO-A1-01/12679
- WO-A1-2004/108799
- WO-A2-2009/032228
- US-A1- 2009 004 579
- Spectra Group Limited: "H-Nu Visible Light & Infrared Photoinitiators", , 15. Juli 2010 (2010-07-15), XP002679388, Gefunden im Internet: URL:http://www.sglinc.com/photoin.htm [gefunden am 2012-07-06]
- Scott Prahl: "Amnthraquinone", , 5. März 2012 (2012-03-05), XP002679389, Gefunden im Internet: URL:http://omlc.ogi.edu/spectra/PhotochemC AD/html/034.html [gefunden am 2012-07-06]

## Beschreibung

Die vorliegende Erfindung betrifft die Verwendung einer Kompositharz-Zusammensetzung sowie ein Verfahren zur stereolithographischen Herstellung dentaler Bauteile wie Inlays, Onlays, Kronen und Brücken auf Basis von Kompositharz.

Unter dem Begriff "Rapid Prototyping" (RP) werden generative Fertigungsverfahren zusammengefasst, bei denen aus computergestützten Konstruktionsdaten (CAD-Daten) 3-dimensionale Modelle oder Bauteile hergestellt werden (A. Gebhardt, Vision of Rapid Prototyping, Ber. DGK 83 (2006) 7-12). Dabei handelt es sich um Verfahren wie Stereolithographie (SL), selektives Lasersintern (SLS), 3D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF) und Direct Ceramic Jet Printing (DCJP), mit denen sich Modelle, Bau- oder Formteile auch in Kleinserie kostengünstig herstellen lassen (A. Gebhardt, Generative Fertigungsverfahren, 3. Aufl., Carl Hanser Verlag, München 2007, 77 ff.). Bei der Stereolithographie handelt es sich um RP-Verfahren (A. Beil, Fertigung von Mikro-Bauteilen mittels Stereolithographie, Düsseldorf 2002, VDI-Verlag 3 ff.), bei denen ein Formteil auf der Basis von CAD-Daten schichtweise aus einem flüssigen und härtbaren Monomerharz aufgebaut wird.

Inlays, Onlays und Provisorien auf Basis von Kompositharzen werden heute üblicherweise aus Blanks (industriell vorgefertigten Blöcken oder Scheiben) geschliffen und dann einzementiert. Die Zementierung dieser an der Oberfläche hoch vernetzten Polymermaterialien erfordert aber einigen Aufwand, da die wenig reaktive Komposit-Oberfläche für eine zuverlässige Haftung am Zahn konditioniert werden muss, beispielsweise durch Sandstrahlen, Konditionierung mit Primern, etc.

Auch stereolithographische Verfahren zur Herstellung dentaler Formkörper auf Basis von Kompositharzen sind an sich bekannt (vgl. Mesaric, Witkowski, Quintessenz Zahntech 2009, 35(9), 1144-1153). Die stereolithographische Fertigung ist insbesondere für die Herstellung dentaler Bauteile von großem Vorteil, da damit eine deutliche Vereinfachung der im zahntechnischen Labor mit großem manuellem Aufwand betriebenen Abform- und Gießprozesse bzw. Fräs- und Schleifoperationen möglich ist und gleichzeitig der bei nicht-generativen Verfahren auftretende große Materialverlust vermieden werden kann. Da heutzutage eine vollständige digitale Prozesskette etabliert ist, lassen sich die klassischen Verfahrensschritte zur Herstellung etwa von mehrgliedrigen Brückengerüsten (Ausrichten im Artikulator, Wachsmodulation, Einbetten und Guss) durch die Digitalisierung des Modells, virtuelle Konstruktion des dentalen Formkörpers und dessen generative stereolithographische Fertigung ersetzen.

Ein stereolithographisches Verfahren zur Herstellung von Dentalimplantaten ist aus der WO 95/28688 bekannt.

WO 97/29901 A1 beschreibt ein Verfahren und ein Gerät zur Herstellung 3-dimensionaler Teile aus einem flüssigen, härtbaren Medium. Dabei werden die Teile schichtenweise aufgebaut, indem jede einzelne Schicht mit einem Laser abgefahren und dabei ausgehärtet wird. Danach wird mittels eines Abstreifers die nächste Schicht des härtbaren Materials aufgetragen und anschließend ebenso gehärtet.

DE 199 38 463 A1 und DE 199 50 284 A1 beschreiben mit sichtbarem Licht härtbare Zusammensetzungen und deren Verwendung zur Herstellung von Dentalrestaurationen aus Kunststoffmaterialen mit RP-Verfahren. Diese erreichen jedoch häufig nicht die für dentale Bauteile wünschenswerte Präzision.

DE 101 14 290 A1 beschreibt die Herstellung von dentalen Formteilen durch 3D-Plotting unter Verwendung von schmelzbaren, kondensierbaren, thermisch oder mit sichtbarem oder insbesondere mit UV-Licht härtbaren, ungefüllten oder gefüllten Materialien.

WO 01/12679 A1 offenbart eine mit sichtbarem Licht aushärtende Zusammensetzung und deren Verwendung in formgebenden Verfahren. Die Zusammensetzung enthält mindestens ein radikalisch polymerisierbares Bindemittel, eine Kombination von Acylphosphinoxid und Fluoron als Initiator und einen Absorber.

WO 2004/108799 A1 offenbart photohärtbare Zusammensetzungen, die mindestens einen oberflächenaktiven Polysiloxanphotoinitiator und optional weitere Photoinitatoren wie zum Beispiel Bis(2,4,6-trimethylbenzoyl)phenylphosphinoxid und einen Absorber enthalten.

WO 2009/032228 A2 offenbart polymerisierbare Materialien zur generativen Herstellung von Dentalrestaurationen, die mindestens einen Photoinitiator, beispielsweise eine Mischung von Campherchinon und 2,4,6-Trimethylbenzoyldiphenylphosphinoxid, und einen Absorber enthalten.

Zusammensetzungen, die einen ersten Photopolymerisationsinitiator mit einem Absorptionsmaximum bei einer Wellenlänge von weniger als 400 nm und eine Monoacyltrialkyl- oder Diacyldialkylgermanium-Verbindung als zweiten Photopolymerisationsinitiator enthalten, werden im Stand der Technik nicht offenbart.

Stereolithographische Verfahren zur Herstellung dentaler Bauteile verwenden üblicherweise Licht mit Wellenlängen im UV-Bereich sowie entsprechende Photoinitiatoren. Daneben werden häufig auch UV-Absorber eingesetzt, die Durchstrahlungs- und Streuungseffekte während des Bauprozesses verringern und so die Präzision der Bauteile verbessern sollen. Stereolithographische Systeme, die ästhetische, zahnfarbene dentale Restaurationen mit genügender Präzision im Wellenlängenbereich von sichtbarem Licht erzeugen, sind dagegen nicht bekannt.

Während des schichtweisen Aufbaus von dentalen Bauteilen mittels stereolithographischer Verfahren werden die polymerisationsfähigen Gruppen in der Regel nur teilweise umgesetzt, so dass die erhaltenen Bauteile eine noch nicht vollständig ausgebildete Festigkeit und Härte aufweisen. Deshalb müssen diese Bauteile nach der stereolithographischen Herstellung und der Reinigung in einem weiteren Schritt nachgehärtet werden. Dies erfolgt beispielsweise durch Bestrahlen und/oder Hitzebehandlung. Dabei reagieren die noch nicht umgesetzten zugänglichen polymerisationsfähigen Gruppen ab und führen so zu erhöhter Festigkeit und Härte. Dieser Schritt führt aber auch dazu, dass die oberflächliche Inhibierungsschicht des Bauteils reduziert wird, was sich negativ auf die Haftung zu einem Zement oder Adhäsivsystem auswirken kann.

Zudem verhindern bei der photochemischen Nachhärtung die eingesetzten Absorber häufig die gewünschte Tiefenwirkung. Für die klinische Performance dentaler Bauteile auf Basis von Kompositharzen ist eine gute Polymerisationstiefe von zentraler Bedeutung. Nach der ISO 4049 - 2009 ("Dentistry - Polymer-based restorative materials") wird die Polymerisationstiefe (depth of cure) so bestimmt, dass ein zylindrischer Kompositprobekörper in einer Stahlform die empfohlene Zeit bestrahlt wird. Dann wird der Probekörper aus der Form genommen und das nichtpolymerisierte Komposit mit einem Kunststoffspatel entfernt. Die Höhe des verbleibenden Zylinders, dividiert durch 2, wird als Polymerisationstiefe bezeichnet und ist quasi ein Maß dafür, wie wirksam das Komposit durch das eingestrahlte Licht ausgehärtet werden kann.

Die Polymerisationstiefe ist sowohl von Prozessparametern als auch von den Materialeigenschaften abhängig. So besteht z.B. zwischen Polymerisationstiefe und der Intensität des eingestrahlten Lichts bzw. der Belichtungszeit ein logarithmischer Zusammenhang (vgl. J. H. Lee, R. K. Prud'homme, I. A. Aksay, J. Mater. Res. 16 (2001) 3536-3544). Dabei sollte das Emissionsspektrum der Strahlungsquelle mit dem Absorptionsspektrum des Photoinitiators gut übereinstimmen. Weiterhin korreliert die Polymerisationstiefe mit der Transluzenz des Komposits, die wiederum u.a. durch den Brechungsindex der Harzmatrix und der Füllstoffe, durch die Größe der Füllstoffpartikel sowie die Art und Konzentration zugesetzter Farbmittel beeinflusst wird (E. Mahn, Zahnmedizin 2011, 50-59). Außerdem wird die Polymerisationstiefe durch die Art und Konzentration des Photoinitiatorsystems beeinflusst, wobei sich monomolekulare Photoinitiatoren einfacher kontrollieren lassen als bimolekulare Photoinitiatorsysteme.

Der Erfindung liegt die Aufgabe zu Grunde, die genannten Nachteile zu vermeiden und ein Verfahren zur Herstellung dentaler Bauteile auf Basis von Kompositharzen mittels Stereolithographie bereitzustellen, das sich durch hohe Bauteilgenauigkeit, gute Durchhärtungstiefe und gute mechanische und ästhetische Eigenschaften der dentalen Bauteile auszeichnet und dabei eine optimale Anbindung an einen Zahn ermöglicht. Insbesondere soll ein Verfahren bereitgestellt werden, bei dem diese vorteilhaften Ergebnisse mit geringem apparativem Aufwand und möglichst wenigen Arbeitsschritten erreicht werden.

Erfindungsgemäß wird diese Aufgabe gelöst durch die Verwendung einer Kompositharz-Zusammensetzung enthaltend
(a) mindestens ein polyreaktives Bindemittel,
(b) einen ersten Photopolymerisationsinitiator mit einem Absorptionsmaximum bei einer Wellenlänge von weniger als 400 nm,
(c) einen zweiten Photopolymerisationsinitiator mit einem Absorptionsmaximum bei einer Wellenlänge von mindestens 400 nm ausgewählt aus der Gruppe bestehend aus Monoacyltrialkyl- und Diacyldialkylgermanium-Verbindungen sowie Mischungen davon und
(d) einen Absorber mit einem Absorptionsmaximum bei einer Wellenlänge von weniger als 400 nm
zur stereolithographischen Herstellung eines dentalen Formteils auf Basis von Kompositharz.

Erfindungsgemäß enthält die Kompositharz-Zusammensetzung allgemein einen ersten Photopolymerisationsinitiator für den UV-Bereich, einen zweiten Photopolymerisationsinitiator für den sichtbaren Bereich und einen UV-Absorber. Es wurde überraschenderweise gefunden, dass die Verwendung einer erfindungsgemäßen Kompositharz-Zusammensetzung die stereolithographische Herstellung dentaler Bauteile auf Basis von Kompositharzen mit hervorragender Präzision, optimaler Durchhärtungstiefe und sehr guten mechanischen Eigenschaften auch in tieferen Schichten ermöglicht.

Die Bestimmung der Wellenlänge des Absorptionsmaximums und des molaren Extinktionskoeffizienten der Photoinitiatoren und Absorber erfolgt üblicherweise mittels UV-VIS-Spektroskopie bei Raumtemperatur unter Verwendung einer Lösung der betreffenden Substanz in einem geeigneten Lösungsmittel wie Acetonitril. Dabei erfolgt die Bestimmung vorzugsweise an Lösungen einer Konzentration von 1 mM. Zur Messung kann beispielsweise ein übliches Zweistrahl-UV/VIS-Spektrometer verwendet werden.

Das langwelligste Absorptionsmaximum des ersten Photopolymerisationsinitiators liegt vorzugsweise bei einer Wellenlänge von weniger als 400 nm, insbesondere im Bereich von 300 bis weniger als 400 nm, bevorzugt im Bereich von 330 bis weniger als 400 nm, besonders bevorzugt im Bereich von 345 bis weniger als 400 nm und am meisten bevorzugt im Bereich von 360 bis weniger als 400 nm.

Das langwelligste Absorptionsmaximum des zweiten Photopolymerisationsinitiators liegt vorzugsweise bei einer Wellenlänge von mindestens 400 nm, insbesondere im Bereich von 400 bis 600 nm, besonders bevorzugt im Bereich von 400 bis 500 nm und am meisten bevorzugt im Bereich von 420 bis 480 nm.

Die Absorptionsspektren des ersten und zweiten Photopolymerisationsinitiators können sich in gewissen Grenzen überschneiden. Vorzugsweise beträgt die Differenz der langwelligsten Absorptionsmaxima des ersten und zweiten Photopolymerisationsinitiators mindestens 5 nm, insbesondere mindestens 10 nm, am meisten bevorzugt mindestens 15 nm. Außerdem ist es bevorzugt, dass der erste Photopolymerisationsinitiator im Wellenlängenbereich von 420 bis 750 nm und insbesondere im Wellenlängenbereich von 440 bis 700 nm einen molaren dekadischen Extinktionskoeffizienten von weniger als 10 l/(mol·cm) aufweist.

Als erfindungsgemäß eingesetzter erster Photopolymerisationsinitiator eignen sich insbesondere Phosphinoxide, Benzoine, Benzilketale, Acetophenone, Benzophenone, Thioxanthone sowie Mischungen davon.

Besonders geeignet sind Acyl- und Bisacylphosphinoxide wie 2,4,6-Trimethylbenzoyldiphenylphosphinoxid oder Bis-(2,4,6-trimethylbenzoyl)phenylphosphinoxid, Benzoin, Benzoinalkylether, Benzildialkylketale wie Benzyldimethylketal, α-Hydroxyacetophenone wie 1-Hydroxy-cyclohexyl-phenyl-keton, 2-Hydroxy-2-methyl-1-phenyl-1-propanon oder 2-Hydroxy-1-[4-(2-hydroxyethoxy)-phenyl]-2-methyl-1-propanon, α-Dialkoxyacetophenone, α-Aminoacetophenone wie 2-Benzyl-2-(dimethylamino)-1-[4-(4-morpholinyl)-phenyl]-1-butanon oder 2-Methyl-1-[4-(methylthio)-phenyl]-2-(4-morpholinyl)-1-propanon, Alkylthioxanthone wie i-Propylthioxanthon sowie Mischungen davon. Acyl- und Bisacylphosphinoxide und insbesondere 2,4,6-Trimethylbenzoyldiphenylphosphinoxid, Bis-(2,4,6-trimethylbenzoyl)-phenylphosphinoxid und deren Mischungen sind besonders bevorzugt.

Als erfindungsgemäß eingesetzter zweiter Photopolymerisationsinitiator eignen sich insbesondere Benzoyltrimethylgermanium, Dibenzoyldiethylgermanium, Bis-(4-methoxybenzoyl)-diethylgermanium und deren Mischungen. Ganz besonders bevorzugt sind auch Mischungen von mindestens einem α-Diketon und mindestens einer Monoacyltrialkyl- oder Diacyldialkylgermanium-Verbindung. Bevorzugte α-Diketone sind Campherchinon, 9,10-Phenanthrenchinon, 1-Phenyl-propan-1,2-dion, Diacetyl, 4,4'-Dichlorbenzil oder deren Derivate.

α-Diketone werden vorzugsweise in Kombination mit Aminbeschleunigern eingesetzt. Als Aminbeschleuniger werden üblicherweise tertiäre Amine verwendet. Geeignet sind insbesondere tertiäre aromatische Amine wie N,N-Dialkyl-aniline, N,N-Dialkyl-p-toluidine oder N,N-Dialkyl-3,5-xylidine, p-(N,N-dialkylamino)-phenylethanole, p-(N,N-dialkylamino)-benzoesäurederivate, p-(N,N-dialkylamino)-benzaldehyde, p-(N,N-dialkylamino)-phenylessigsäureester oder p-(N,N-dialkylamino)-phenylpropionsäureester. Konkrete Beispiele hierfür sind N,N-Dimethylanilin, N,N-Dimethyl-p-toluidin, 3,5,N,N-Tetramethylanilin, p-(N,N-Dimethylamino)-benzaldehyd, p-(Dimethylamino)-benzoesäureethylester und p-(Dimethylamino)-benzonitril sowie Mischungen davon. Geeignet sind auch tertiäre aliphatische Amine wie Tri-n-butylamin, 2-Dimethylaminoethanol, Triethanolamin, Dimethylaminoethylmethacrylat, N,N-Dimethylbenzylamin, heterocyclische Amine wie 1,2,2,6,6-Pentamethylpiperidin, Aminosäure-Derivate wie N-Phenylglycin sowie Mischungen davon. p-(Dimethylamino)-benzoesäureethylester, Dimethylaminoethylmethacrylat, N,N-Dimethylanilin, N,N-Dimethyl-p-toluidin, Triethanolamin und deren Mischungen sind besonders bevorzugt. Dabei sind insbesondere solche Photopolymerisationsinitiatoren bevorzugt, welche bei der Einbringung von Strahlung, deren Emissionsmaximum bei einer Wellenlänge von mindestens 400 nm liegt, ausbleichen und so nach der weiteren Aushärtung keine störende Eigenfärbung mehr besitzen. Dies trifft insbesondere auf die genannten Acylgermanium-Verbindungen zu.

In einer bevorzugten Ausführungsform wird als zweiter Photopolymerisationsinitiator eine Mischung von mindestens einer Monoacyltrialkyl- und Diacyldialkylgermanium-Verbindung mit mindestens einem α-Diketon in Kombination mit mindestens einem Aminbeschleuniger verwendet. Ganz besonders bevorzugte Kombinationen dieser Photopolymerisationsinitiatoren sind in der parallelen Anmeldung EP 12163823.3 beschrieben.

Die erfindungsgemäß eingesetzte Kompositharz-Zusammensetzung enthält ferner vorzugsweise mindestens einen Absorber, dessen langwelligstes Absorptionsmaximum bei einer Wellenlänge von weniger als 400 nm, insbesondere im Bereich von 300 bis weniger als 400 nm, bevorzugt im Bereich von 330 bis weniger als 400 nm, besonders bevorzugt im Bereich von 345 bis weniger als 400 nm und am meisten bevorzugt im Bereich von 360 bis weniger als 400 nm liegt. Als Absorber eignen sich insbesondere Benzotriazole, Triazine, Benzophenone, Cyanoacrylate, Salicylsäurederivate, Hindered Amine Light Stabilizers (HALS) sowie Mischungen davon. Ebenfalls als Absorber geeignet sind anorganische Salze wie nanoskalige Titandioxide und Zinkoxide. Weiterhin sind Absorber bevorzugt, die eine Löslichkeit von mindestens 0,2 Gew.-% und insbesondere mindestens 0,5 Gew.-% in der Kompositharz-Zusammensetzung aufweisen.

Besonders geeignet sind o-Hydroxyphenylbenzotriazole wie 2-(2*H-*benzotriazol-2-yl)-4-methylphenol, 2-(5-Chlor-2*H*-benzotriazol-2-yl)-4-methyl-6-*tert*-butyl-phenol, 2-(5-Chlor-2*H*-benzotriazol-2-yl)-4,6-di-*tert*-butyl-phenol, 2-(2*H*-Benzotriazol-2-yl)-4,6-di-*tert*-pentyl-phenol, 2-(2*H*-Benzotriazol-2-yl)-4-methyl-6-dodecylphenol, 2-(2*H*-Benzotriazol-2-yl)-4,6-bis-(1-methyl-1-phenylethyl)-phenol, 2-(2*H*-Benzotriazol-2-yl)-6-(1-methyl-1-phenylethyl)-4-(1,1,3,3-tetramethylbutyl)-phenol, 2-(2*H*-Benzotriazol-2-yl)-4-(1,1,3,3-tetramethylbutyl)-phenol oder 3-(2*H*-benzotriazol-2-yl)-5-*tert*-butyl-4-hydroxy-benzolpropansäureester, o-Hydroxyphenyltriazine wie 2-(2-Hydroxy-4-hexyloxy-phenyl)-4,6-diphenyl-1,3,5-triazin oder 2-(2-Hydroxy-4-[2-hydroxy-3-dodecyloxy-propyloxy]-phenyl)-4,6-bis-(2,4-dimethylphenyl)-1,3,5-triazin, o-Hydroxybenzophenone wie 2-Hydroxy-4-octyloxy-benzophenon, Cyanoacrylate wie Ethyl-2-cyano-3,3-diphenylacrylat, 2-Ethylhexyl-2-cyano-3,3-diphenylacrylat oder Tetrakis-[(2-cyano-3,3-diphenylacryloyl)oxymethyl]-methan, Hindered Amine Light Stabilizers (HALS) wie N,N'-Bisformyl-N,N'-bis-(2,2,6,6-tetramethyl-4-piperidinyl)-hexamethylendiamin, Bis-(2,2,6,6-tetramethyl-4-piperidyl)-sebacate, Bis-(1,2,2,6,6-pentamethyl-4-piperidyl)-sebacate oder Methyl-(1,2,2,6,6-pentamethyl-4-piperidyl)-sebacate, Salicylsäureester sowie Mischungen davon. o-Hydroxyphenylbenzotriazole und insbesondere 2-(2*H-*Benzotriazol-2-yl)-4-methyl-6-dodecylphenol sind besonders bevorzugt.

Dabei sind erfindungsgemäß solche Absorber bevorzugt, die kaum oder gar nicht im Wellenlängenbereich des zweiten Photopolymerisationsinitiators absorbieren. Insbesondere sind Absorber bevorzugt, die im Wellenlängenbereich von 400 bis 750 nm und insbesondere im Wellenlängenbereich von 420 bis 750 nm und am meisten bevorzugt im Wellenlängenbereich von 440 bis 700 nm einen molaren dekadischen Extinktionskoeffizienten von weniger als 10 l/(mol·cm) aufweisen.

In einer besonders bevorzugten Ausführungsform wird als erster Photopolymerisationsinitiator mindestens ein Phosphinoxid, als zweiter Photopolymerisationsinitiator eine Mischung von mindestens einer Monoacyltrialkyl- und Diacyldialkylgermanium-Verbindung mit mindestens einem α-Diketon in Kombination mit mindestens einem Aminbeschleuniger und als Absorber mindestens ein Benzotriazol verwendet. Ein konkretes Beispiel ist eine Kombination von Bis-(2,4,6-trimethylbenzoyl)-phenylphosphinoxid, Bis-(4-methoxybenzoyl)-diethylgermanium, Campherchinon, 4-(N,N-Dimethylamino)-benzoesäurediethylester und 2-(2*H*-Benzotriazol-2-yl)-4-methyl-6-dodecylphenol.

Die Kompositharz-Zusammensetzung enthält mindestens ein polyreaktives Bindemittel. Bevorzugt sind Bindemittel auf Basis radikalisch polymerisierbarer Monomere und/oder Prepolymere.

Als radikalisch polymerisierbare Bindemittel eignen sich besonders mono- oder multifunktionelle (Meth)acrylate oder deren Mischungen. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 polymerisierbaren Gruppen verstanden. Geeignete Beispiele sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA (ein Additionsprodukt aus Methacrylsäure und Bisphenol-A-diglycidylether), ethoxy- oder propoxyliertes Bisphenol-A-Dimethacrylat wie z.B. das Bisphenol-A-Dimethacrylat SR-348c (Sartomer) mit 3 Ethoxygruppen oder 2,2-Bis[4-(2-methacryloxypropoxy)phenyl]propan, UDMA (ein Additionsprodukt aus 2-Hydroxyethylmethacrylat und 2,2,4-Trimethylhexamethylendiisocyanat), Di-, Tri- oder Tetraethylenglycol-di(meth)acrylat, Trimethylolpropantri(meth)acrylat, Pentaerythrittetra(meth)acrylat, sowie Glycerindi- und trimethacrylat, 1,4-Butandioldi(meth)acrylat, 1,10-Decandioldi(meth)acrylat (D3MA) oder 1,12-Dodecandioldi(meth)acrylat. Bevorzugte (Meth)acrylatmonomere sind Benzyl-, Tetrahydrofurfuryl- oder Isobornylmethacrylat, p-Cumyl-phenoxyethylenglycolmethacrylat, 2,2-Bis[4-(2-methacryloxypropoxy)phenyl]propan, Bis-GMA, UDMA, SR-348c und D3MA.

Als radikalisch polymerisierbares Bindemittel lassen sich auch N-mono- oder N-disubstitiuierte Acrylamide wie z.B. N-Ethylacrylamid oder N,N-Dimethacrylamid oder Bisacrylamide wie z.B. N,N'-Diethyl-1,3-bis(acrylamido)propan, 1,3-Bis(methacrylamido)-propan, 1,4-Bis(acrylamido)butan oder 1,4-Bis(acryloyl)piperazin einsetzen.

Weiterhin lassen sich als radikalisch polymerisierbares Bindemittel auch bekannte schrumpfungsarme radikalisch ringöffnend polymerisierbare Monomere, wie z.B. mono- oder multifunktionelle Vinylcyclopropane bzw. bicyclische Cyclopropanderivate (vgl. DE 196 16 183 C2 bzw. EP 1 413 569 A1), oder cyclische Allylsulfide (vgl. US 6,043,361 oder US 6,344,556) einsetzen, die darüber hinaus auch in Kombination mit den vorstehend aufgeführten Di(meth)acrylat-Vernetzern verwendet werden können.

Außerdem können als radikalisch polymerisierbares Bindemittel auch radikalisch polymerisierbare Polysiloxane eingesetzt werden, die aus geeigneten Methacrylsilanen wie z.B. 3-(Methacryloyloxy)propyltrimethoxysilan hergestellt werden können und z.B. in der DE 199 03 177 C2 beschrieben sind.

Vorzugsweise werden Mischungen der vorstehend genannten Monomere verwendet.

Weiterhin enthält die erfindungsgemäß eingesetzte Kompositharz-Zusammensetzung zur Verbesserung der mechanischen Eigenschaften oder zur Einstellung der Viskosität vorzugsweise auch organisehe oder anorganische Füllstoffpartikel. Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgröße von 0,005 bis 2 µm, vorzugsweise 0,1 bis 1 µm, nanopartikuläre oder mikrofeine Füllstoffe wie pyrogene Kieselsäure oder Fällungskieselsäure mit einer mittleren durchschnittlichen Partikelgröße von 5 bis 200 nm, vorzugsweise 10 bis 100 nm, Minifüllstoffe wie Quarz-, Glaskeramik- oder Glaspulver mit einer durchschnittlichen Teilchengröße von 0,01 bis 10 µm, vorzugsweise 0,1 bis 1 µm, sowie röntgenopake Füllstoffe wie Ytterbiumtrifluorid oder nanopartikuläres Tantal(V)-oxid bzw. Bariumsulfat mit einer mittleren durchschnittlichen Partikelgröße von 10 bis 1000 nm, vorzugsweise 100 bis 300 nm.

Außerdem können die erfindungsgemäß eingesetzten Zusammensetzungen weitere Additive enthalten, vor allem Lösungsmittel wie Wasser oder Ethanol bzw. entsprechende Lösungsmittelgemische, sowie beispielsweise Stabilisatoren, Aromastoffe, Farbmittel, mikrobiozide Wirkstoffe, fluoridionenabgebende Additive, optische Aufheller oder Weichmacher.

Besonders bevorzugt sind Kompositharz-Zusammensetzungen, die die folgenden Bestandteile enthalten:
(a) 5 bis 90 Gew.-%, insbesondere 10 bis 40 Gew.-% und besonders bevorzugt 20 bis 40 Gew.-% polyreaktives Bindemittel,
(b) 0,01 bis 5,0 Gew.-%, insbesondere 0,1 bis 3,0 Gew.-% und besonders bevorzugt 0,1 bis 1,0 Gew.-% ersten Photopolymerisationsinitiator,
(c) 0,01 bis 2,0 Gew.-%, insbesondere 0,1 bis 1,0 Gew.-% und besonders bevorzugt 0,1 bis 0,5 Gew.-% zweiten Photopolymerisationsinitiator,
(d) 0,001 bis 3,0 Gew.-%, insbesondere 0,1 bis 2,0 Gew.-% und besonders bevorzugt 0,5 bis 1,0 Gew.-% Absorber und
(e) 5 bis 90 Gew.-%, insbesondere 40 bis 90 Gew.-% und besonders bevorzugt 50 bis 80 Gew.-% Füllstoff,
jeweils bezogen auf die Gesamtmasse der Zusammensetzung.

Ganz besonders bevorzugt sind Kompositharz-Zusammensetzungen, die die folgenden Bestandteile enthalten:
(a) 10 bis 40 Gew.-% polyreaktives Bindemittel,
(b) 0,1 bis 3,0 Gew.-% ersten Photopolymerisationsinitiator,
(c) 0,1 bis 1,0 Gew.-% zweiten Photopolymerisationsinitiator,
(d) 0,1 bis 2,0 Gew.-% Absorber und
(e) 40 bis 90 Gew.-% Füllstoff,
jeweils bezogen auf die Gesamtmasse der Zusammensetzung.

Die Erfindung betrifft auch ein Verfahren zur stereolithographischen Herstellung eines dentalen Formteils, bei dem eine Kompositharz-Zusammensetzung wie oben definiert schichtweise durch lokales Einbringen von Strahlung unter Ausbildung eines dreidimensionalen Körpers gehärtet wird.

Insbesondere betrifft die Erfindung auch ein Verfahren zur stereolithographischen Herstellung eines dentalen Formteils, bei dem
(i) eine Kompositharz-Zusammensetzung wie oben definiert schichtweise durch lokales Einbringen von Strahlung, deren Emissionsmaximum bei einer Wellenlänge von weniger als 400 nm liegt, unter Ausbildung eines dreidimensionalen Körpers gehärtet wird und
(ii) der erhaltene dreidimensionale Körper durch Einbringen von Strahlung, deren Emissionsmaximum bei einer Wellenlänge von mindestens 400 nm liegt, weiter ausgehärtet wird.

Dabei werden üblicherweise in Schritt (i) die polymerisierbaren Gruppen des polyreaktiven Bindemittels nur teilweise umgesetzt und somit das Kompositharz nur teilweise ausgehärtet. Anschließend kann optional eine Reinigung des erhaltenen dreidimensionalen Körpers erfolgen, bei der beispielsweise überschüssiges Kompositmaterial mechanisch entfernt wird. Schließlich wird der erhaltene dreidimensionale Körper in Schritt (ii) weiter ausgehärtet.

Im Vergleich zur Herstellung von dentalen Bauteilen aus Kompositmaterialien mittels Schleiftechniken zeichnet sich das erfindungsgemäße stereolithographische Verfahren durch hohe Wirtschaftlichkeit aus, da die Bauzeiten im Vergleich zu den Schleifzeiten von Blanks sehr gering gehalten werden können und eine Parallelfertigung möglich ist. Außerdem wird im Gegensatz zu geschliffenen Blanks nur sehr wenig Material für den Bauprozess benötigt. Ein weiterer Vorteil besteht darin, dass mit dem erfindungsgemäßen stereolithographischen Verfahren dentale Bauteile wie Inlays und Onlays mit deutlich feineren und besser ausgeprägten Oberflächenstrukturen wie Okklusalflächen und Fissuren hergestellt werden können, die durch Schleiftechniken nicht zugänglich sind.

Es hat sich zudem überraschend gezeigt, dass durch die erfindungsgemäße Verwendung mindestens eines zweiten Photopolymerisationsinitiators sowie von Strahlung mit einem Emissionsmaximum im sichtbaren Bereich bei der weiteren Aushärtung in Schritt (ii) eine vollständige Härtung mit optimaler Tiefenwirkung erreicht wird.

Das langwelligste Emissionsmaximum der in Schritt (i) verwendeten Strahlung liegt vorzugsweise bei einer Wellenlänge von weniger als 400 nm, insbesondere im Bereich von 240 bis weniger als 400 nm und am meisten bevorzugt im Bereich von 320 bis weniger als 400 nm. Das langwelligste Emissionsmaximum der in Schritt (ii) verwendeten Strahlung liegt vorzugsweise bei einer Wellenlänge von mindestens 400 nm, insbesondere im Bereich von 400 bis 600 nm und am meisten bevorzugt im Bereich von 400 bis 500 nm.

Die weitere Aushärtung in Schritt (ii) kann beispielsweise in einem Lichtofen (z.B. Lumamat®) durchgeführt werden. Dabei wird vorzugsweise eine Lichtintensität von mindestens 1 mW/cm², insbesondere mindestens 10 mW/cm² und besonders bevorzugt mindestens 100 mW/cm² verwendet.

Die Erfindung betrifft auch die Verwendung einer Kompositharz-Zusammensetzung wie oben definiert in einem erfindungsgemäßen Verfahren.

Die weitere Aushärtung in Schritt (ii) kann auch intraoral erfolgen. Dies ist erfindungsgemäß besonders bevorzugt. Dabei besteht der Vorteil, dass kein zusätzliches Vergütungsgerät oder separater Arbeitsschritt benötigt wird.

Die Erfindung betrifft daher auch eine Kompositharz-Zusammensetzung wie oben definiert zur Verwendung in einem Verfahren zur dentalen Restauration, bei dem
(i) eine Kompositharz-Zusammensetzung wie oben definiert schichtweise durch lokales Einbringen von Strahlung, deren Emissionsmaximum bei einer Wellenlänge von weniger als 400 nm liegt, unter Ausbildung eines dreidimensionalen Körpers gehärtet wird und
(ii) der erhaltene dreidimensionale Körper in den Mund eines Patienten eingebracht und durch Einbringen von Strahlung, deren Emissionsmaximum bei einer Wellenlänge von mindestens 400 nm liegt, weiter ausgehärtet wird.

Bevorzugte Ausführungsformen hinsichtlich der Kompositharz-Zusammensetzung und der verwendeten Strahlung sind wie oben definiert.

Die intraorale weitere Aushärtung kann beispielsweise mit einer dentalen LED-Lichtquelle (z.B. Bluephase, Ivoclar Vivadent AG, Extinktionsmaximum 460 nm) erfolgen. Dabei wird vorzugsweise eine Lichtintensität von mindestens 1 mW/cm², insbesondere mindestens 10 mW/cm² und besonders bevorzugt mindestens 100 mW/cm² verwendet.

Besonders bevorzugt wird in Schritt (ii) zunächst eine Schicht eines Dentalzements zur Zementierung des dentalen Bauteils am Zahn aufgebracht und diese bei der weiteren Aushärtung in Schritt (ii) mit ausgehärtet. Dabei kann aufgrund des Vorhandenseins von noch nicht umgesetzten polymerisierbaren Gruppen insbesondere auf der Oberfläche des dreidimensionalen Körpers eine optimale Anbindung des Komposits an den Zement erzielt werden. Dies bringt große Sicherheit und ist äußerst anwenderfreundlich.

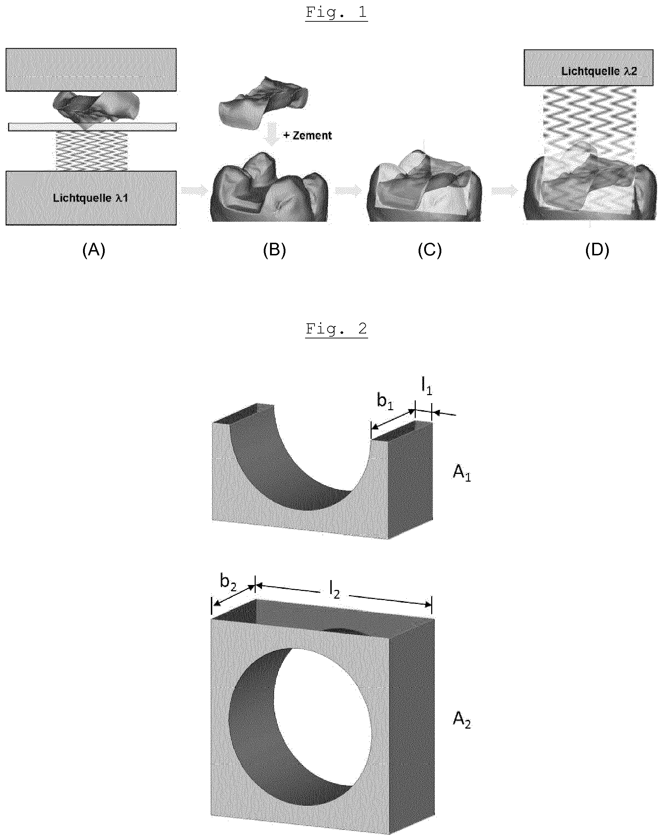

Ein solches Verfahren ist in Fig. 1 schematisch dargestellt. Dabei wird zunächst ein dreidimensionaler Körper in der Form eines dentalen Bauteils, beispielsweise einer Krone, mittels Stereolithographie erzeugt, indem eine Kompositharz-Zusammensetzung schichtweise durch lokales Einbringen von Strahlung einer Lichtquelle λ1, deren Emissionsmaximum bei einer Wellenlänge von weniger als 400 nm liegt, unter Ausbildung des dreidimensionalen Körpers gehärtet wird (A). Der so erhaltene dreidimensionale Körper wird dann zusammen mit einer Schicht eines Dentalzements auf einen Zahn aufgebracht (B und C). Anschließend wird durch Einbringen von Strahlung einer Lichtquelle λ2, deren Emissionsmaximum bei einer Wellenlänge von mindestens 400 nm liegt, der dreidimensionale Körper weiter ausgehärtet und zugleich durch Aushärtung des Dentalzements am Zahn zementiert (D).

Die erfindungsgemäß verwendete Kompositharz-Zusammensetzung und das erfindungsgemäße Verfahren eignen sich besonders zur stereolithographischen Herstellung von dreidimensionalen Körpern und dentalen Bauteilen in Form von Inlays, Onlays, Kronen und Brücken auf Basis von Kompositharzen.

Die Erfindung wird im Folgenden anhand von Beispielen näher erläutert.

### Beispiele

### Beispiel 1

Es wurden Harze der folgenden Zusammensetzungen hergestellt:

| | Harz A (Vergleich) | Harz B |
|---|---|---|
| Komponente | Gew.-% | Gew.-% |
| UDMA¹⁾ | 14,20 | 14,33 |
| Bis-GMA²) | 14,70 | 13,84 |
| Decan-1,10-dioldimethacrylat | 7,35 | 7,17 |
| Campherchinon | 0,09 | 0,09 |
| Aminbeschleuniger³⁾ | 0,22 | 0,21 |
| Lucerin TPO | 0,15 | 0,15 |
| Ivocerin | 0,04 | 0,04 |
| Dispergieradditiv⁴⁾ | 0,25 | 0,24 |
| anorganische Füllstoffmischung⁵⁾ | 63,00 | 61,43 |
| Tinuvin 571 | - | 2,50 |

| | | |
|---|---|---|
| ¹⁾ Additionsprodukt aus 2-Hydroxyethylmethacrylat und 2,2,4-Trimethylhexamethylendiisocyanat ²⁾ Additionsprodukt aus Methacrylsäure und Bisphenol-A-diglycidylether ³⁾ 4-Dimethylaminobenzoesäureethylester ⁴⁾ Säuregruppenhaltiges Dispergieradditiv (Byk-Chemie) ⁵⁾ Mischung aus pyrogener Kieselsäure, Bariumaluminiumsilikat-Glaspulver und Ytterbiumfluorid im Gewichtsverhältnis 3:2:1 | | |

Unter Verwendung der jeweiligen Harze und Strahlung einer Wellenlänge von 388 nm wurden stereolithographisch Körper mit der in Fig. 2 gezeigten Geometrie hergestellt, wobei die Soll-Kantenlängen l₁ = 1 mm, l₂ = 10 mm und b₁ = b₂ = 5 mm betrugen. Zur Bestimmung der Geometriegenauigkeit wurden die tatsächlichen Kantenlängen der erhaltenen Körpers ausgemessen.

Bei dem aus dem Harz A hergestellten Körper betrug die absolute Abweichung von der Sollgeometrie an jeder Kante etwa 600 µm. Die Abweichung der Fläche A₁ = l₁*b₁ betrug etwa 95 %, und die Abweichung der Fläche A₂ = l₂*b₂ betrug etwa 38 %.

Dagegen betrug bei dem aus dem erfindungsgemäßen Harz B hergestellten Körper die absolute Abweichung von der Sollgeometrie an keiner Kante mehr als 90 µm. Die Abweichung der Fläche A₁ = l₁*b₁ betrug lediglich etwa 10 %, und die Abweichung der Fläche A₂ = l₂*b₂ betrug lediglich etwa 5 %. Dies entspricht einer Verbesserung der Geometriegenauigkeit um etwa 90 % und zeigt, dass durch Verwendung des erfindungsgemäßen Harzes eine hervorragende Geometriegenauigkeit erzielt werden kann.

### Beispiel 2

Unter Verwendung des Harzes B aus Beispiel 1 wurden stereolithographisch 10 zylindrische Probekörper (Durchmesser 4 mm, Höhe 4 mm) hergestellt, die auf Metallplättchen gemäß ISO 10477:2004 (D) unter Verwendung des Haftsystems SR Link und des Befestigungskomposits Variolink 2 aufgebracht und mittels Belichtung mit einer LED Lichtquelle Bluephase, Programm HiP (Ivoclar Vivadent AG) mit Licht einer Wellenlänge von 460 nm 3 x 10 s von oben durch den Prüfkörper belichtet wurden. Dadurch konnte gleichzeitig eine Aushärtung des Befestigungskomposits und eine Nachhärtung der stereolithographisch hergestellten Körper erzielt werden. Es wurde eine mittlere Scherhaftfestigkeit von 17,58 MPa gemessen.

### Beispiel 3

Es wurde ein Harz der folgenden Zusammensetzung hergestellt:

| Komponente | Gew.-% |
|---|---|
| Bis-GMA¹⁾ | 15,26 |
| UDMA²⁾ | 14,43 |
| Decan-1,10-dioldimethacrylat | 7,07 |
| Campherchinon | 0,09 |
| Aminbeschleuniger³⁾ | 0,21 |
| Lucerin TPO | 0,14 |
| farbgebende Pigmente | 0,43 |
| Inhibitor | 0,03 |
| anorganische Füllstoffmischung⁴⁾ | 62,04 |
| Tinuvin 571 | 0,30 |

| | |
|---|---|
| ¹⁾ Additionsprodukt aus Methacrylsäure und Bisphenol-A-diglycidylether ²⁾ Additionsprodukt aus 2-Hydroxyethylmethacrylat und 2,2,4-Trimethylhexamethylendiisocyanat ³⁾ 4-Dimethylaminobenzoesäureethylester ⁴⁾ Mischung aus pyrogener Kieselsäure, Bariumaluminiumsilikat-Glaspulver und Ytterbiumfluorid im Gewichtsverhältnis 3:2:1 | |

Unter Verwendung dieses Harzes und Strahlung einer Wellenlänge von 388 nm wurden stereolithographisch 10 Probekörper (Breite 2 mm, Länge 2 mm, Höhe 25 mm) hergestellt. Die 3-Punkt-Biegefestigkeit und das Biege-E-Modul der so erhaltenen Körper wurden gemäß dem ISO-Standard ISO-4049 (Dentistry - Polymer-based filling, restorative and luting materials) bestimmt. Es wurden eine mittlere Biegefestigkeit von 62,4 MPa und ein mittleres E-Modul von 1695 N/mm² gemessen.

10 weitere auf dieselbe Weise hergestellte Probekörper wurden im Anschluss an den stereolithographischen Bauprozess unter Verwendung einer LED Lichtquelle Bluephase, Programm HiP (Ivoclar Vivadent AG) mit Licht einer Wellenlänge von 460 nm 3 x 10 s einseitig nachvergütet. Es wurden eine mittlere Biegefestigkeit von 84,2 MPa und ein mittleres E-Modul von 2466 N/mm² gemessen.

## Patentansprüche

1. Verwendung einer Kompositharz-Zusammensetzung enthaltend
(a) mindestens ein polyreaktives Bindemittel,
(b) einen ersten Photopolymerisationsinitiator mit einem Absorptionsmaximum bei einer Wellenlänge von weniger als 400 nm,
(c) einen zweiten Photopolymerisationsinitiator mit einem Absorptionsmaximum bei einer Wellenlänge von mindestens 400 nm ausgewählt aus der Gruppe bestehend aus Monoacyltrialkyl- und Diacyldialkylgermanium-Verbindungen sowie Mischungen davon und
(d) einen Absorber mit einem Absorptionsmaximum bei einer Wellenlänge von weniger als 400 nm
zur stereolithographischen Herstellung eines dentalen Formteils auf Basis von Kompositharz.

2. Verwendung nach Anspruch 1, bei der das langwelligste Absorptionsmaximum des ersten Photopolymerisationsinitiators bei einer Wellenlänge von weniger als 400 nm und insbesondere bei einer Wellenlänge im Bereich von 300 bis weniger als 400 nm, bevorzugt im Bereich von 330 bis weniger als 400 nm, besonders bevorzugt im Bereich von 345 bis weniger als 400 nm und ganz besonders bevorzugt im Bereich von 360 bis weniger als 400 nm liegt und/oder das langwelligste Absorptionsmaximum des zweiten Photopolymerisationsinitiators bei einer Wellenlänge im Bereich von 400 bis 600 nm, bevorzugt im Bereich von 400 bis 500 nm und am meisten bevorzugt im Bereich von 420 bis 480 nm liegt.

3. Verwendung nach Anspruch 1 oder 2, bei der das langwelligste Absorptionsmaximum des Absorbers bei einer Wellenlänge von weniger als 400 nm, insbesondere bei einer Wellenlänge im Bereich von 300 bis weniger als 400 nm, bevorzugt im Bereich von 330 bis weniger als 400 nm, besonders bevorzugt im Bereich von 345 bis weniger als 400 nm und am meisten bevorzugt im Bereich von 360 bis weniger als 400 nm liegt und/oder die Differenz der Absorptionsmaxima des ersten und zweiten Photopolymerisationsinitiators mindestens 5 nm, insbesondere mindestens 10 nm und am meisten bevorzugt mindestens 15 nm beträgt.

4. Verwendung nach einem der Ansprüche 1 bis 3, bei der die Zusammensetzung einen ersten Photopolymerisationsinitiator ausgewählt aus der Gruppe bestehend aus Phosphinoxiden, Benzoinen, Benzilketalen, Acetophenonen, Benzophenonen, Thioxanthonen sowie Mischungen davon, insbesondere ausgewählt aus der Gruppe bestehend aus Acyl- und Bisacylphosphinoxiden, Benzoin, Benzoinalkylethern, Benzildialkylketalen, α-Hydroxyacetophenonen, α-Dialkoxyacetophenonen, α-Aminoacetophenonen, Alkylthioxanthonen sowie Mischungen davon und am meisten bevorzugt ausgewählt aus der Gruppe bestehend aus 2,4,6-Trimethylbenzoyldiphenylphosphinoxid, Bis-(2,4,6-trimethylbenzoyl)-phenylphosphinoxid sowie Mischungen davon enthält.

5. Verwendung nach einem der Ansprüche 1 bis 4, bei der die Zusammensetzung einen zweiten Photopolymerisationsinitiator ausgewählt aus der Gruppe bestehend aus Benzoyltrimethylgermanium, Dibenzoyldiethylgermanium, Bis-(4-methoxybenzoyl)diethylgermanium sowie Mischungen davon enthält.

6. Verwendung nach einem der Ansprüche 1 bis 5, bei der die Zusammensetzung einen Absorber ausgewählt aus der Gruppe bestehend aus Benzotriazolen, Triazinen, Benzophenonen, Cyanoacrylaten, Salicylsäurederivaten, Hindered Amine Light Stabilizers (HALS), anorganischen Salzen sowie Mischungen davon, insbesondere ausgewählt aus der Gruppe bestehend aus o-Hydroxyphenylbenzotriazolen, o-Hydroxyphenyltriazinen, o-Hydroxybenzophenonen, Cyanoacrylaten, Hindered Amine Light Stabilizers (HALS), Salicylsäureestern, nanoskaligen Titandioxiden und Zinkoxiden sowie Mischungen davon und am meisten bevorzugt 2-(2*H*-Benzotriazol-2-yl)-4-methyl-6-dodecylphenol enthält.

7. Verwendung nach einem der Ansprüche 1 bis 6, bei der das mindestens eine polyreaktive Bindemittel aus radikalisch polymerisierbaren Monomeren und Prepolymeren und insbesondere aus mono- und multifunktionellen (Meth)acrylaten und deren Mischungen ausgewählt ist.

8. Verwendung nach einem der Ansprüche 1 bis 7, bei der die Zusammensetzung ferner Füllstoff enthält.

9. Verwendung nach einem der Ansprüche 1 bis 8, bei der die Zusammensetzung
(a) 5 bis 90 Gew.-% polyreaktives Bindemittel,
(b) 0,01 bis 5,0 Gew.-% ersten Photopolymerisationsinitiator,
(c) 0,01 bis 2,0 Gew.-% zweiten Photopolymerisationsinitiator,
(d) 0,001 bis 3,0 Gew.-% Absorber und
(e) 5 bis 90 Gew.-% Füllstoff enthält,
jeweils bezogen auf die Gesamtmasse der Zusammensetzung.

10. Verwendung nach einem der Ansprüche 1 bis 9, bei der das dentale Formteil die Form eines Inlays, Onlays, einer Krone oder einer Brücke hat.

11. Verfahren zur Herstellung eines dentalen Formteils mittels Stereolithographie, bei dem
(i) eine Kompositharz-Zusammensetzung enthaltend
(a) mindestens ein polyreaktives Bindemittel,
(b) einen ersten Photopolymerisationsinitiator mit einem Absorptionsmaximum bei einer Wellenlänge von weniger als 400 nm,
(c) einen zweiten Photopolymerisationsinitiator mit einem Absorptionsmaximum bei einer Wellenlänge von mindestens 400 nm und
(d) einen Absorber mit einem Absorptionsmaximum bei einer Wellenlänge von weniger als 400 nm
und vorzugsweise wie in einem der Ansprüche 1 bis 10 definiert schichtweise durch lokales Einbringen von Strahlung, deren Emissionsmaximum bei einer Wellenlänge von weniger als 400 nm liegt, unter Ausbildung eines dreidimensionalen Körpers gehärtet wird und
(ii) der erhaltene dreidimensionale Körper durch Einbringen von Strahlung, deren Emissionsmaximum bei einer Wellenlänge von mindestens 400 nm liegt, weiter ausgehärtet wird.

12. Verwendung einer Kompositharz-Zusammensetzung enthaltend
(a) mindestens ein polyreaktives Bindemittel,
(b) einen ersten Photopolymerisationsinitiator mit einem Absorptionsmaximum bei einer Wellenlänge von weniger als 400 nm,
(c) einen zweiten Photopolymerisationsinitiator mit einem Absorptionsmaximum bei einer Wellenlänge von mindestens 400 nm und
(d) einen Absorber mit einem Absorptionsmaximum bei einer Wellenlänge von weniger als 400 nm
und vorzugsweise wie in einem der Ansprüche 1 bis 10 definiert in einem Verfahren gemäß Anspruch 11.

13. Kompositharz-Zusammensetzung enthaltend
(a) mindestens ein polyreaktives Bindemittel,
(b) einen ersten Photopolymerisationsinitiator mit einem Absorptionsmaximum bei einer Wellenlänge von weniger als 400 nm,
(c) einen zweiten Photopolymerisationsinitiator mit einem Absorptionsmaximum bei einer Wellenlänge von mindestens 400 nm und
(d) einen Absorber mit einem Absorptionsmaximum bei einer Wellenlänge von weniger als 400 nm
und vorzugsweise wie in einem der Ansprüche 1 bis 10 definiert zur Verwendung in einem Verfahren zur dentalen Restauration, bei dem
(i) die Kompositharz-Zusammensetzung schichtweise durch lokales Einbringen von Strahlung, deren Emissionsmaximum bei einer Wellenlänge von weniger als 400 nm liegt, unter Ausbildung eines dreidimensionalen Körpers gehärtet wird und
(ii) der erhaltene dreidimensionale Körper in den Mund eines Patienten eingebracht und durch Einbringen von Strahlung, deren Emissionsmaximum bei einer Wellenlänge von mindestens 400 nm liegt, weiter ausgehärtet wird.

14. Kompositharz-Zusammensetzung zur Verwendung nach Anspruch 13, bei der in Schritt (ii) zunächst eine Schicht eines Dentalzements zur Zementierung des dentalen Bauteils am Zahn aufgebracht und diese bei der weiteren Aushärtung in Schritt (ii) mit ausgehärtet wird.

15. Kompositharz-Zusammensetzung zur Verwendung nach Anspruch 13 oder 14, wobei der dreidimensionale Körper die Form eines Inlays, Onlays, einer Krone oder einer Brücke hat.

## Claims

1. Use of a composite resin composition comprising
(a) at least one polyreactive binder,
(b) a first photopolymerisation initiator having an absorption maximum at a wavelength of less than 400 nm,
(c) a second photopolymerisation initiator having an absorption maximum at a wavelength of at least 400 nm selected from the group consisting of monoacyltrialkyl- and diacyldialkylgermanium compounds and mixtures thereof and
(d) an absorber having an absorption maximum at a wavelength of less than 400 nm,
for the stereolithographic production of a dental moulded part based on composite resin.

2. Use according to claim 1, in which the longest-wavelength absorption maximum of the first photopolymerisation initiator is at a wavelength of less than 400 nm and in particular at a wavelength in the range of 300 to less than 400 nm, preferably in the range of 330 to less than 400 nm, particularly preferably in the range of 345 to less than 400 nm and quite particularly preferably in the range of 360 to less than 400 nm and/or the longest-wavelength absorption maximum of the second photopolymerisation initiator is at a wavelength in the range of 400 to 600 nm, preferably in the range of 400 to 500 nm and most preferably in the range of 420 to 480 nm.

3. Use according to claim 1 or 2, in which the longest-wavelength absorption maximum of the absorber is at a wavelength of less than 400 nm, in particular at a wavelength in the range of 300 to less than 400 nm, preferably in the range of 330 to less than 400 nm, particularly preferably in the range of 345 to less than 400 nm and most preferably in the range of 360 to less than 400 nm and/or the difference between the absorption maxima of the first and second photopolymerisation initiators is at least 5 nm, in particular at least 10 nm and most preferably at least 15 nm.

4. Use according to one of claims 1 to 3, in which the composition comprises a first photopolymerisation initiator selected from the group consisting of phosphine oxides, benzoins, benzil ketals, acetophenones, benzophenones, thioxanthones as well as mixtures thereof, in particular selected from the group consisting of acyl- and bisacylphosphine oxides, benzoin, benzoin alkyl ethers, benzil dialkyl ketals, α-hydroxyacetophenones, α-dialkoxyacetophenones, α-aminoacetophenones, alkylthioxanthones as well as mixtures thereof and most preferably selected from the group consisting of 2,4,6-trimethylbenzoyl diphenylphosphine oxide, bis-(2,4,6-trimethylbenzoyl)phenylphosphine oxide as well as mixtures thereof.

5. Use according to one of claims 1 to 4, in which the composition comprises a second photopolymerisation initiator selected from the group consisting of benzoyltrimethylgermanium, dibenzoyldiethylgermanium, bis-(4-methoxybenzoyl)diethylgermanium as well as mixtures thereof.

6. Use according to one of claims 1 to 5, in which the composition comprises an absorber selected from the group consisting of benzotriazoles, triazines, benzophenones, cyanoacrylates, salicylic acid derivatives, hindered amine light stabilisers (HALS), inorganic salts as well as mixtures thereof, in particular selected from the group consisting of o-hydroxyphenylbenzotriazoles, o-hydroxyphenyltriazines, o-hydroxybenzophenones, cyanoacrylates, hindered amine light stabilisers (HALS), salicylic acid esters, nanoscale titanium dioxides and zinc oxides as well as mixtures thereof and most preferably comprises 2-(2*H-*benzotriazol-2-yl)-4-methyl-6-dodecylphenol.

7. Use according to one of claims 1 to 6, in which the at least one polyreactive binder is selected from radically polymerisable monomers and prepolymers and in particular from mono- and multifunctional (meth)acrylates and their mixtures.

8. Use according to one of claims 1 to 7, in which the composition further comprises filler.

9. Use according to one of claims 1 to 8, in which the composition comprises
(a) 5 to 90 wt % polyreactive binder,
(b) 0.01 to 5.0 wt % first photopolymerisation initiator,
(c) 0.01 to 2.0 wt % second photopolymerisation initiator,
(d) 0.001 to 3.0 wt % absorber and
(e) 5 to 90 wt % filler,
in each case relative to the total mass of the composition.

10. Use according to one of claims 1 to 9, in which the dental moulded part has the shape of an inlay, onlay, a crown or a bridge.

11. Process for producing a dental moulded part by means of stereolithography, in which
(i) a composite resin composition comprising
(a) at least one polyreactive binder,
(b) a first photopolymerisation initiator having an absorption maximum at a wavelength of less than 400 nm,
(c) a second photopolymerisation initiator having an absorption maximum at a wavelength of at least 400 nm and
(d) an absorber having an absorption maximum at a wavelength of less than 400 nm
and preferably, as defined in one of claims 1 to 10, the composite resin composition is cured in layers by the local introduction of radiation, the emission maximum of which is at a wavelength of less than 400 nm, to form a three-dimensional body, and
(ii) the resulting three-dimensional body is further cured by introducing radiation, the emission maximum of which is at a wavelength of at least 400 nm.

12. Use of a composite resin composition comprising
(a) at least one polyreactive binder,
(b) a first photopolymerisation initiator having an absorption maximum at a wavelength of less than 400 nm,
(c) a second photopolymerisation initiator having an absorption maximum at a wavelength of at least 400 nm and
(d) an absorber having an absorption maximum at a wavelength of less than 400 nm
and preferably, as defined in one of claims 1 to 10, in a process according to claim 11.

13. Composite resin composition comprising
(a) at least one polyreactive binder,
(b) a first photopolymerisation initiator having an absorption maximum at a wavelength of less than 400 nm,
(c) a second photopolymerisation initiator having an absorption maximum at a wavelength of at least 400 nm and
(d) an absorber having an absorption maximum at a wavelength of less than 400 nm and preferably, as defined in one of claims 1 to 10, for the use in a process for dental restoration, in which
(i) the composite resin composition is cured in layers by the local introduction of radiation, the emission maximum of which is at a wavelength of less than 400 nm, to form a three-dimensional body, and
(ii) the resulting three-dimensional body is introduced into the mouth of a patient and further cured by introducing radiation, the emission maximum of which is at a wavelength of at least 400 nm.

14. Composite resin composition for use according to claim 13, in which in step (ii) a layer of a dental cement is initially applied to cement the dental component to the tooth, and this layer is also cured during the further curing in step (ii).

15. Composite resin composition for use according to claim 13 or 14, wherein the three-dimensional body has the shape of an inlay, onlay, a crown or a bridge.

## Revendications

1. Utilisation d'une composition de résine composite contenant :
a) au moins un liant polyréactif,
b) un premier amorceur de photopolymérisation présentant un maximum d'absorption à une longueur d'onde de moins de 400 nm,
c) un deuxième amorceur de photopolymérisation présentant un maximum d'absorption à une longueur d'onde d'au moins 400 nm, et choisi dans l'ensemble formé par les composés de type monoacyltrialkyl-germanium ou diacyl-dialkyl-germanium et les mélanges de tels composés,
d) et un absorbeur présentant un maximum d'absorption à une longueur d'onde de moins de 400 nm,
pour la fabrication par stéréolithographie d'une pièce dentaire façonnée à base de résine composite.

2. Utilisation conforme à la revendication 1, dans laquelle le maximum d'absorption de plus grande longueur d'onde du premier amorceur de photopolymérisation se trouve à une longueur d'onde de moins de 400 nm et en particulier à une longueur d'onde située dans le domaine allant de 300 à moins de 400 nm, de préférence dans le domaine allant de 330 à moins de 400 nm, mieux encore dans le domaine allant de 345 à moins de 400 nm, et surtout dans le domaine allant de 360 à moins de 400 nm, et/ou le maximum d'absorption de plus grande longueur d'onde du deuxième amorceur de photopolymérisation se trouve à une longueur d'onde située dans le domaine allant de 400 à 600 nm, de préférence dans le domaine allant de 400 à 500 nm, et surtout dans le domaine allant de 420 à 480 nm.

3. Utilisation conforme à la revendication 1 ou 2, dans laquelle le maximum d'absorption de plus grande longueur d'onde de l'absorbeur se trouve à une longueur d'onde de moins de 400 nm et en particulier à une longueur d'onde située dans le domaine allant de 300 à moins de 400 nm, de préférence dans le domaine allant de 330 à moins de 400 nm, mieux encore dans le domaine allant de 345 à moins de 400 nm, et surtout dans le domaine allant de 360 à moins de 400 nm, et/ou la différence entre les maximums d'absorption des premier et deuxième amorceurs de photopolymérisation vaut au moins 5 nm, en particulier au moins 10 nm, et surtout au moins 15 nm.

4. Utilisation conforme à l'une des revendications 1 à 3, dans laquelle la composition contient un premier amorceur de photopolymérisation choisi dans l'ensemble formé par les oxydes de phosphine, benzoïnes, cétals de benzile, acétophénones, benzophénones et thioxanthones et les mélanges de tels composés, choisi en particulier dans l'ensemble formé par les oxydes d'acyl-phosphine ou de bisacyl-phosphine, la benzoïne, les alkyl-éthers de benzoïne, les dialkyl-cétals de benzile, les α-hydroxy-acétophénones, les α-dialcoxy-acétophénones, les α-amino-acétophénones, les alkyl-thioxanthones et les mélanges de tels composés, et choisi surtout dans l'ensemble formé par l'oxyde de (2,4,6-triméthyl-benzoyl)-diphényl-phosphine, l'oxyde de bis(2,4,6-triméthyl-benzoyl)-phényl-phosphine, et les mélanges de ces composés.

5. Utilisation conforme à l'une des revendications 1 à 4, dans laquelle la composition contient un deuxième amorceur de photopolymérisation choisi dans l'ensemble formé par le benzoyl-triméthyl-germanium, le dibenzoyl-diéthyl-germanium, le bis(4-méthoxy-benzoyl)-diéthyl-germanium, et les mélanges de ces composés.

6. Utilisation conforme à l'une des revendications 1 à 5, dans laquelle la composition contient un absorbeur choisi dans l'ensemble formé par les benzotriazoles, triazines, benzophénones, cyanoacrylates, dérivés de l'acide salicylique, photo-stabilisants de type amine encombrée ou « HALS » et sels inorganiques et les mélanges de tels composés, et choisi en particulier dans l'ensemble formé par les (o-hydroxy-phényl)-benzotriazoles, les (o-hydroxy-phényl)-triazines, les o-hydroxy-benzophénones, les cyano-acrylates, les photo-stabilisants HALS, les esters de l'acide salicylique, et des nanoparticules de dioxyde de titane ou d'oxyde de zinc, et les mélanges de tels composés, et surtout, contient du 2-(2*H*-benzotriazol-2-yl)-4-méthyl-6-dodécyl-phénol.

7. Utilisation conforme à l'une des revendications 1 à 6, dans laquelle le liant polyréactif, au nombre d'au moins un, est choisi parmi les monomères et prépolymères polymérisables par voie radicalaire, et en particulier, parmi les acrylates et méthacrylates monofonctionnels ou polyfonctionnels et les mélanges de tels composés.

8. Utilisation conforme à l'une des revendications 1 à 7, dans laquelle la composition contient en outre une charge.

9. Utilisation conforme à l'une des revendications 1 à 8, dans laquelle la composition contient :
a) de 5 à 90 % en poids de liant polyréactif,
b) de 0,01 à 5,0 % en poids du premier amorceur de photopolymérisation,
c) de 0,01 à 2,0 % en poids du deuxième amorceur de photopolymérisation,
d) de 0,001 à 3,0 % en poids d'absorbeur,
e) et de 5 à 90 % en poids de charge,
chacun de ces pourcentages étant rapporté au poids total de la composition.

10. Utilisation conforme à l'une des revendications 1 à 9, dans laquelle la pièce dentaire façonnée a la forme d'une prothèse inlay ou onlay, d'une couronne ou d'un bridge.

11. Procédé de fabrication d'une pièce dentaire façonnée par stéréolithographie, dans lequel
i) on fait durcir par couches, par application locale d'un rayonnement dont le maximum d'émission se trouve à une longueur d'onde de moins de 400 nm, une composition de résine composite contenant :
a) au moins un liant polyréactif,
b) un premier amorceur de photopolymérisation présentant un maximum d'absorption à une longueur d'onde de moins de 400 nm,
c) un deuxième amorceur de photopolymérisation présentant un maximum d'absorption à une longueur d'onde d'au moins 400 nm,
d) et un absorbeur présentant un maximum d'absorption à une longueur d'onde de moins de 400 nm,
et de préférence telle que définie dans l'une des revendications 1 à 10, en formant ainsi un corps tridimensionnel,
ii) et l'on poursuit le durcissement du corps tridimensionnel obtenu, par application d'un rayonnement dont le maximum d'émission se trouve à une longueur d'onde d'au moins 400 nm.

12. Utilisation d'une composition de résine composite contenant :
a) au moins un liant polyréactif,
b) un premier amorceur de photopolymérisation présentant un maximum d'absorption à une longueur d'onde de moins de 400 nm,
c) un deuxième amorceur de photopolymérisation présentant un maximum d'absorption à une longueur d'onde d'au moins 400 nm,
d) et un absorbeur présentant un maximum d'absorption à une longueur d'onde de moins de 400 nm,
et de préférence telle que définie dans l'une des revendications 1 à 10, dans un procédé conforme à la revendication 11.

13. Composition de résine composite contenant :
a) au moins un liant polyréactif,
b) un premier amorceur de photopolymérisation présentant un maximum d'absorption à une longueur d'onde de moins de 400 nm,
c) un deuxième amorceur de photopolymérisation présentant un maximum d'absorption à une longueur d'onde d'au moins 400 nm,
d) et un absorbeur présentant un maximum d'absorption à une longueur d'onde de moins de 400 nm,
et de préférence telle que définie dans l'une des revendications 1 à 10, pour utilisation dans un procédé de restauration dentaire dans lequel
i) on fait durcir la composition de résine composite par couches, par application locale d'un rayonnement dont le maximum d'émission se trouve à une longueur d'onde de moins de 400 nm, en formant ainsi un corps tridimensionnel,
ii) et l'on place dans la bouche d'un patient le corps tridimensionnel obtenu et l'on en poursuit le durcissement par application d'un rayonnement dont le maximum d'émission se trouve à une longueur d'onde d'au moins 400 nm.

14. Composition de résine composite pour utilisation conforme à la revendication 13, dans laquelle, dans l'étape (ii), on applique d'abord sur la dent une couche d'un ciment dentaire pour fixer la pièce dentaire et l'on fait durcir cette couche avec lors du durcissement complémentaire de l'étape (ii).

15. Composition de résine composite pour utilisation conforme à la revendication 13 ou 14, dans laquelle le corps tridimensionnel a la forme d'une prothèse inlay ou onlay, d'une couronne ou d'un bridge.
